# FASCICULE DE BREVET EUROPEEN

(11) **EP 3 384 000 B1**
(45) Date de publication et mention de la délivrance du brevet: **10.08.2022**
(21) Numéro de dépôt: 16805090.4
(22) Date de dépôt: 30.11.2016
(51) Int. Cl.: C12N 1/12, A23K 10/16

(54) **PROCÉDÉ DE CULTURE D'ALGUES ROUGES UNICELLULAIRES (ARU) AVEC DU PERMÉAT LACTÉ**
VERFAHREN ZUR KULTIVIERUNG VON EINZELLIGEN ROTEN ALGEN (URA) MIT MILCHPERMEAT
METHOD FOR CULTURING UNICELLULAR RED ALGAE (URA) WITH MILK PERMEATE

(30) Priorité: 04.12.2015 FR 1561839
(43) Date de publication de la demande: 10.10.2018
(73) Titulaire: Fermentalg, 33500 Libourne (FR)
(72) Inventeur: CAGNAC, Olivier, 33500 Libourne (FR); GEHIN, Bruno, 33330 Saint Emilion (FR); CHAMPEAU, Marion, 33550 Langoiran (FR)
(74) Mandataire: Be IP
(86) Numéro de dépôt international: PCT/EP2016/079325
(87) Numéro de publication internationale: WO 2017/093345

(56) Documents cités:
- EP-A1- 2 730 647
- WO-A2-2014/074769
- GILBERT TISCHENDORF ET AL: "Ultrastructure and enzyme complement of proplastids from heterotrophically grown cells of the red alga Galdieria sulphuraria", EUROPEAN JOURNAL OF PHYCOLOGY, vol. 42, no. 3, 1 août 2007 (2007-08-01), pages 243-251, XP055279073, DE ISSN: 0967-0262, DOI: 10.1080/09670260701437642
- WOLFGANG GROSS ET AL: "Heterotrophic Growth of Two Strains of the Acido-Thermophilic Red Alga Galdieria sulphuraria", PLANT AND CELL PHYSIOLOGY, OXFORD UNIVERSITY PRESS, UK, vol. 36, no. 4, 1 janvier 1995 (1995-01-01), pages 633-638, XP009180796, ISSN: 0032-0781
- OLAV SUNE GRAVERHOLT ET AL: "Heterotrophic high-cell-density fed-batch and continuous-flow cultures of Galdieria sulphuraria and production of phycocyanin", APPLIED MICROBIOLOGY AND BIOTECHNOLOGY, SPRINGER, BERLIN, DE, vol. 77, no. 1, 5 septembre 2007 (2007-09-05), pages 69-75, XP019560681, ISSN: 1432-0614, DOI: 10.1007/S00253-007-1150-2
- FABIAN BUMBAK ET AL: "Best practices in heterotrophic high-cell-density microalgal processes: achievements, potential and possible limitations", APPLIED MICROBIOLOGY AND BIOTECHNOLOGY, SPRINGER, BERLIN, DE, vol. 91, no. 1, 13 mai 2011 (2011-05-13), pages 31-46, XP019915707, ISSN: 1432-0614, DOI: 10.1007/S00253-011-3311-6
- RIKKE ANKERSTJERNE SCHMIDT ET AL: "Heterotrophic high cell-density fed-batch cultures of the phycocyanin-producing red algaGaldieria sulphuraria", BIOTECHNOLOGY AND BIOENGINEERING, vol. 90, no. 1, 5 avril 2005 (2005-04-05), pages 77-84, XP055114003, ISSN: 0006-3592, DOI: 10.1002/bit.20417
- G. BARBIER: "Comparative Genomics of Two Closely Related Unicellular Thermo-Acidophilic Red Algae, Galdieria sulphuraria and Cyanidioschyzon merolae, Reveals the Molecular Basis of the Metabolic Flexibility of Galdieria sulphuraria and Significant Differences in Carbohydrate Metabolism of Both Algae", PLANT PHYSIOLOGY, vol. 137, no. 2, 1 février 2005 (2005-02-01), pages 460-474, XP055345493, Rockville, Md, USA ISSN: 0032-0889, DOI: 10.1104/pp.104.051169
- HENKANATTE-GEDERA S M ET AL: "Algal-based, single-step treatment of urban wastewaters", BIORESOURCE TECHNOLOGY, vol. 189, 6 avril 2015 (2015-04-06), pages 273-278, XP029221608, ISSN: 0960-8524, DOI: 10.1016/J.BIORTECH.2015.03.120

## Description

### DOMAINE DE L'INVENTION

La présente invention concerne le domaine de la culture des algues rouges unicellulaires (ARUs). L'invention s'adresse particulièrement à un procédé de culture d'ARUs du genre Galdieria, caractérisé en ce que le milieu de culture comprend du perméat lacté comme principal apport d'au moins une source de carbone.

### ETAT DE LA TECHNIQUE

Certaines algues rouges unicellulaires (ARUs) peuvent être intéressantes comme source additionnelle de nourriture, car elles peuvent être source de protéines, comme par exemple des phycobiliprotéines, ou encore de fibres, de lipides, ou encore d'agents antioxydants particulièrement des caroténoïdes. Elles peuvent être utilisées natives, avantageusement séchées, mais aussi transformées, par exemple réduites sous forme de farines. Elles peuvent être utilisées, de manière non thérapeutique, dans l'alimentation humaine ou animale, comme supplément nutritionnel ou incorporées en petite quantité dans des aliments.

Les algues rouges, ou Rhodophytes (division des Rhodophyta), sont un grand taxon d'algues pour la plupart marines. Il comprend des organismes unicellulaires (ARUs) mais aussi multicellulaires. Les algues rouges sont caractérisées par une composition pigmentaire avec de la chlorophylle "a", des caroténoïdes et des pigments caractéristiques, les phycobiliprotéines.

Les ARUs sont des organismes extrémophiles capables de supporter des pH très acides (0,05-5), ainsi que des températures très élevées (25-56°C).

Les ARUs sont cultivés sur des milieux de culture standard et selon leur mode de culture, peuvent utiliser comme source de carbone le dioxyde de carbone gazeux ou bien une source de carbone organique comme les sucres, les acides organiques ou leurs sels, ou les alcools ou polyols. (Wolfgang Gross and Claus Schnarrenberger, 1995).

Certains documents citent le lactose parmi de nombreuses sources de carbone pour la culture de microorganismes (WO 2014/074769), et notamment pour les microalgues appartenant au genre *Isochrysis* (WO 2012/175866). Tischendorf & al. (2007), dans une étude des plastides de *Galdieria,* une ARU, décrivent sa culture de faible densité en Erlen dans un milieu comprenant du lactose. Le lactose est également décrit parmi de nombreux sucres comme agent antifloculant pour éviter l'autofloculation de microalgues lyophilisées (EP 2 730 647).

En dehors de ces exemples, le lactose n'est pas identifié ni même employé comme source de carbone dans la culture industrielle de microalgues. Le coût de la matière première et la difficulté de s'approvisionner en quantités suffisantes à un prix économiquement viable font partie des raisons de cette absence d'usage, alors que de nombreuses sources de carbone moins coûteuses sont disponibles en grande quantité.

Le lactose est un glucide naturellement présent dans le lait des mammifères. C'est un diholoside dont la molécule est composée de glucose et de galactose. C'est le seul glucide du lait. A titre d'exemple le lait de vache en contient 5g/100ml. Le lactosérum, ou petit lait, obtenu après séparation des matières grasses et précipitation de la caséine, en est très riche, de 70 % à 75 % des matières sèches.

Le perméat de lait obtenu après extraction des protéines du lait par ultrafiltration peut contenir, après concentration par osmose inverse pour en séparer l'eau et cristallisation jusqu'à environ 86% de lactose.

Le perméat de sérum venant de la filière fromagère a une composition similaire à celle du perméat de lait, de même que le babeurre qui lui est issu de la production du beurre.

Les perméats lactés en général, particulièrement celui de lait, issus de l'industrie laitière sont des coproduits difficilement valorisables par les industriels. Les volumes de perméat produits dans cette industrie sont très importants et il n'est pas facile pour les industriels de trouver un débouché économique pour ce produit, ou alors comme source coûteuse de lactose après concentration, cristallisation du lactose, et séchage, pour entrer dans la composition de certains laits reconstitués. Les perméats liquides ne trouvent pas de débouchés acceptables et sont peu propice à la culture de microorganismes susceptibles de les dégrader du fait de la présence d'acide lactique et d'acide citrique.

On comprend donc qu'il existe un besoin en débouchés permettant la valorisation des perméats lactés qu'ils soient de lait, de sérum ou du babeurre.

Les inventeurs ont montré que les algues rouges unicellulaires (ARUs) du genre Galdieria, présentent la capacité de croitre dans un milieu comprenant un perméat lacté comme principal apport d'au moins une source de carbone, ladite source de carbone comprenant essentiellement du lactose.

Cette invention permet à la fois de valoriser un déchet de l'industrie laitière et de produire une biomasse riche en protéines et en composants à haute valeur ajoutée comme des pigments pouvant être utilisés dans l'industrie alimentaire.

### EXPOSÉ DE L'INVENTION

La présente invention concerne un procédé de production d'une biomasse d'algues rouges unicellulaires (ARUs) du genre Galdieria comprenant les étapes suivantes :
a) de culture des dites algues rouges unicellulaires (ARUs) du genre Galdieria dans un milieu comprenant au moins un perméat lacté comprenant du lactose comme source de carbone, du lactate et du citrate.
b) de récupération de la biomasse produite comprenant lesdites ARUs du genre Galdieria à partir du milieu de culture.

Par milieu comprenant au moins un perméat lacté comprenant du lactose comme source de carbone on entend dans le présent texte un milieu de culture de microorganismes, en particulier de microalgues, dont tout ou partie de la source de carbone est sous la forme de lactose en une quantité comprise entre 0,05 g/L et 200 g/L, avantageusement entre 1 g/L et 150g/L, très avantageusement entre 10 g/L et 80 g/L, ledit lactose provenant du perméat lacté.

Selon un mode particulier de réalisation de l'invention, le procédé de culture comprend au moins une étape d'éclairage.

L'invention concerne également la biomasse susceptible d'être obtenue par ce procédé.

### DESCRIPTION DÉTAILLÉE DE L'INVENTION

La présente invention a pour objet premier un procédé de culture d'algues rouges unicellulaires (ARUs) du genre Galdieria, dans un milieu comprenant du lactose comme source de carbone sous la forme de perméat lacté, en particulier de perméat de lait, de perméat de sérum ou de babeurre, c'est à dire un milieu de culture comprenant au moins un perméat lacté comprenant du lactose comme source de carbone.

Selon un premier mode de réalisation de l'invention, le lactose est la seule source de carbone dans le milieu de culture. Dans ce cas le milieu de culture pourrait comprendre des traces d'autres matières carbonées susceptibles d'être dégradées par les ARUs, ces traces provenant du lactose ou bien d'autres composants entrant dans la composition du milieu de culture. On citera notamment les acides organiques comme par exemple l'acide lactique et l'acide citrique, contenus dans le perméat lacté, qui sont également une source de carbone.

Par « lactose comme seule source de carbone dans le milieu de culture » on entend que le milieu de culture ne comprend pas de sources de carbone ajoutées au perméat lacté.

Selon un autre mode de réalisation de l'invention, le milieu de culture peut comprendre une autre source de carbone employée de manière usuelle pour la culture de microorganismes comme le glucose, le saccharose et l'acétate.

De manière préférentielle, les éléments contenus dans les perméats lactés (lactose, acides organiques, protéines) sont les seules sources de carbone du milieu de culture.

Selon l'invention encore, le lactose peut être à une concentration initiale dans le milieu de culture comprise entre 0,05 g/L, voire 0,1 g/L et 200 g/L, préférentiellement comprise entre 1 g/L, voire 5 g/L et 100 g/L, plus préférentiellement 10 g/L et 30 g/L voire 80 g/L. L'homme du métier connaissant la teneur du lactose dans le perméat lacté qu'il aura à sa disposition, saura déterminer la quantité de perméat à employer pour obtenir la concentration en lactose désirée.

Par perméat lacté, on entend selon l'invention le perméat de lait, le perméat de sérum ou le babeurre, de préférence le perméat de lait. Ces perméats comprenent généralement de 80 à 250 g/L de lactose.

Selon l'invention tout milieu de culture de microalgues, particulièrement d'algues rouges unicellulaires (ARUs) de la classe des *Cyanidiophyceae,* décrit dans l'art antérieur peut être adapté afin que tout ou partie de la source de carbone qu'il comprend soit sous la forme de lactose, pour être utilisé dans le procédé selon l'invention.

Par la suite dans le présent texte, par commodité et sous réserve de précision, l'emploi de "ARU" doit s'entendre comme signifiant "Rhodophytes, en particulier de la sous-division des Cyanidiophytina, plus particulièrement de la classe des Cyanidiophyceae, notamment de l'ordre des Cyanidiales, des familles des Cyanidiaceae ou des Galdieriaceae, avantageusemennt des genres *Cyanidioschyzon, Cyanidium* ou *Galdieria,* des espèces *Cyanidioschyzon merolae* 10D, *Cyanidioschyzon merolae* DBV201, *Cyanidium caldarium, Cyanidium daedalum, Cyanidium maximum, Cyanidium partitum, Cyanidium rumpens, Galdieria daedala, Galdieria maxima, Galdieria partita* ou encore *Galdieria sulphuraria*".

De même l'emploi du terme "Cyanidiophyceae" doit être compris, sous réserve de précision, comme signifiant "Cyanidiophyceae, de l'ordre des Cyanidiales, des familles des Cyanidiaceae ou des Galdieriaceae, des genres *Cyanidioschyzon, Cyanidium* ou *Galdieria,* des espèces *Cyanidioschyzon merolae* 10D, *Cyanidioschyzon merolae* DBV201, *Cyanidium caldarium, Cyanidium daedalum, Cyanidium maximum, Cyanidium partitum, Cyanidium rumpens, Galdieria daedala, Galdieria maxima, Galdieria partita* ou encore *Galdieria sulphuraria*" ; l'emploi du terme "Cyanidiales" doit être compris, sous réserve de précision, comme signifiant "Cyanidiales, des familles des Cyanidiaceae ou des Galdieriaceae, des genres *Cyanidioschyzon, Cyanidium* ou *Galdieria,* des espèces *Cyanidioschyzon merolae* 10D, *Cyanidioschyzon merolae* DBV201, *Cyanidium caldarium, Cyanidium daedalum, Cyanidium maximum, Cyanidium partitum, Cyanidium rumpens, Galdieria daedala, Galdieria maxima, Galdieria partita* ou encore *Galdieria sulphuraria*" ; l'emploi de l'expression "Cyanidiaceae ou des Galdieriaceae" doit être compris, sous réserve de précision, comme signifiant "Cyanidiaceae ou des Galdieriaceae, des genres *Cyanidioschyzon, Cyanidium* ou *Galdieria,* des espèces *Cyanidioschyzon merolae* 10D, *Cyanidioschyzon merolae* DBV201, *Cyanidium caldarium, Cyanidium daedalum, Cyanidium maximum, Cyanidium partitum, Cyanidium rumpens, Galdieria daedala, Galdieria maxima, Galdieria partita* ou encore *Galdieria sulphuraria*" ; l'emploi de l'expression " *Cyanidioschyzon, Cyanidium* ou *Galdieria* ", doit être compris, sous réserve de précision, comme signifiant " *Cyanidioschyzon, Cyanidium* ou *Galdieria,* des espèces *Cyanidioschyzon merolae* 10D, *Cyanidioschyzon merolae* DBV201, *Cyanidium caldarium, Cyanidium daedalum, Cyanidium maximum, Cyanidium partitum, Cyanidium rumpens, Galdieria daedala, Galdieria maxima, Galdieria partita* ou encore *Galdieria sulphuraria*".

De préférence, les ARUs sont choisies parmi les familles des Cyanidiaceae ou des Galdieriaceae, particulièrement des genres *Cyanidioschyzon, Cyanidium* ou *Galdieria,* très particulièrement des espèces *Cyanidioschyzon merolae* 10D, *Cyanidioschyzon merolae* DBV201, *Cyanidium caldarium, Cyanidium daedalum, Cyanidium maximum, Cyanidium partitum, Cyanidium rumpens, Galdieria daedala, Galdieria maxima, Galdieria partita* ou encore *Galdieria sulphuraria,* préférentiellement de l'espèce *Galdieria sulphuraria.*

Selon l'invention, la culture peut être réalisée par toute technique de culture connue, par exemple en fioles ou en réacteur, mais aussi en fermenteurs ou encore dans tout contenant apte à la croissance de ARUs du genre Galdieria comme par exemple des bassins de type "raceway/openpond", à condition que ladite technique permette de mettre en contact les ARUs avec au moins la source carbonée comprenant au moins du lactose, et le cas échéant équipée d'au moins une source de lumière.

Un autre avantage du procédé mis au point par la Demanderesse est qu'il peut être conduit en bioréacteur, qui plus est avantageusement à l'échelle industrielle. De manière avantageuse, le procédé selon l'invention est mis en œuvre dans des bioréacteurs de 1 m³, 4 m³, 10 m³ et 200 m³, ou plus qui sont des volumes couramment utilisés en production industrielle.

A la connaissance de la demanderesse aucune production industrielle (en bioréacteur) d'ARUs dans un milieu comprenant un perméat lacté comprenant du lactose comme source de carbone n'est connue.

Certaines ARUs sont des êtres mixotrophes, capable à la fois d'hétérotrophie (consommation de substrat organique carboné apporté par le milieu de culture) et d'autotrophie (utilisation de la lumière pour capter le CO₂ via la photosynthèse). La mixotrophie est également appelée photo-hétérotrophie. La notion de mixotrophie est étendue à l'utilisation de la lumière non seulement pour la photosynthèse mais également comme signal lumineux pouvant induire une réponse du métabolisme : par exemple la synthèse de pigments.

La mixotrophie à dominante hétérotrophe permet de produire des molécules d'origine algale en couplant à la fois les avantages de l'autotrophie et de l'hétérotrophie. Elle consiste à introduire une composante lumineuse pouvant être de faible intensité et/ou de courte durée, avec un milieu de culture pouvant contenir une ou plusieurs sources organiques de carbone. Comme en hétérotrophie, les ARUs consomment un substrat organique, ce qui permet d'atteindre une productivité (exprimée en gramme de biomasse sèche/L/h) et/ou une concentration en biomasse (exprimée en gramme de biomasse sèche/L) importante, le chloroplaste et les autres structures sensibles à la lumière de la cellule étant alors activés.

Ces capteurs d'énergie lumineuse peuvent être des organites ou structures spécifiques au sein de la cellule tels que le chloroplaste, le stigma, le chronoplaste, le chromoplaste ou le phycobilisome, ou peuvent être des molécules individuelles capables de répondre à la lumière et d'entraîner une réponse cellulaire comme les rhodopsines, les phytochromes, les cryptochromes ou les auréochromes.

Ces photorécepteurs permettent d'augmenter la productivité de la cellule ainsi que de permettre la synthèse de toutes les molécules pouvant être métabolisées par une ARU. Les molécules d'intérêt, produites par lesdites ARUs, peuvent présenter un intérêt industriel majeur particulièrement dans les domaines de la nutrition, de la cosmétique, de la chimie verte et de l'énergie.

Ces molécules d'intérêt sont variées telles que par exemple des carbohydrates, des protéines, des acides aminés, avantageusement des acides aminés essentiels et des pigments, particulièrement des pigments photosynthétiques, dont les principaux sont les chlorophylles, les caroténoïdes et les phycobiliprotéines.

La Demanderesse a également montré, de manière surprenante et après de longues recherches, qu'une culture d'ARUs du genre Galdieria dans un milieu de culture comprenant au moins un perméat lacté comprenant du lactose comme source de carbone et au moins une étape d'éclairage, avantageusement un éclairage par une lumière présentant un spectre étroit centré sur une longueur d'onde donnée, particulièrement un spectre centré à 455 nm, peut permettre d'obtenir une biomasse dont la quantité en ARUs peut être nettement améliorée par rapport aux cultures habituellement réalisées et qui plus est pouvant contenir une quantité de molécules d'intérêt, particulièrement des phycobiliprotéines particulièrement de la phycocyanine, de l'allophycocyanine, de la phycoérythrine, de la zéaxanthine et des ß-carotènes, éventuellement supérieure à celle pouvant être obtenue dans les cultures décrites dans l'art antérieur.

L'invention concerne donc également un procédé de culture d'algues rouges unicellulaires (ARUs) du genre Galdieria, , dans un milieu comprenant au moins un perméat lacté comprenant du lactose comme source de carbone, ledit procédé comprenant au moins une étape d'éclairage. Ce procédé permet d'obtenir une productivité et/ou une concentration en biomasse nettement améliorée par rapport aux cultures habituellement réalisées, biomasse qui en outre peut être avantageusement riche en phycocyanine et/ou en caroténoïdes.

Par "riche en phycocyanine et/ou en caroténoïdes" on entend dans le présent texte que la concentration en phycobiliprotéines et/ou de caroténoïdes dans la biomasse pouvant être produite après culture dans les conditions selon l'invention est plus importante que la concentration de phycobiliprotéines et/ou de caroténoïdes pouvant être produite par une biomasse des mêmes algues obtenue après culture dans les conditions décrites dans l'art antérieur.

La biomasse obtenue comprend avantageusement des teneurs intracellulaires en phycobiliprotéines (majoritairement la phycocyanine et allophycocyanine) comprises entre 29 et 250 mg/g de matière sèche, préférentiellement entre 35 et 150 mg/g de matière sèche et éventuellement riche en agents antioxydants en particulier en caroténoïdes, avantageusement de la zéaxanthine et du β-carotène, en teneurs comprises entre 0,1 et 10 mg/g, avantageusement entre 0,250 et 1 mg/g.

Selon l'invention, l'étape d'éclairage du procédé peut être réalisée à l'aide d'une lumière blanche ou, avantageusement, d'une lumière bleue ou d'une lumière violette. Préférentiellement selon une variante de l'invention l'étape d'éclairage du procédé peut être réalisée à l'aide d'une lumière bleue.

Selon l'invention, par lumière bleue on entend un rayonnement présentant un spectre étroit de longueur d'onde comprise entre 400 et 550 nm, préférentiellement entre 420 nm et 500 nm. Un tel rayonnement peut permettre d'obtenir une biomasse riche en phycocyanine et en agents antioxydants, particulièrement en caroténoïdes. Selon une variante de l'invention ledit spectre peut être centré à 455 nm et ne pas s'étendre au-delà de 25 nm de chaque côté. De préférence selon l'invention, la longueur d'onde choisie sera comprise entre 430 et 480 nm, très préférentiellement la longueur d'onde choisie sera de 455 nm.

Selon l'invention, l'éclairage peut être produit par tout moyen connu de l'homme du métier, notamment une ou plusieurs lampes, un ou plusieurs tubes, une ou plusieurs diodes électroluminescentes (DELs).

La Demanderesse a démontré que le procédé est encore plus efficace lorsque l'éclairage est réalisé par une ou plusieurs diode(s) électroluminescente(s) (DEL). Ainsi, selon une variante de l'invention, l'éclairage peut être réalisé par une ou plusieurs DELs. Les DELs sont de préférence des DELs du commerce. A titre d'exemple on citera les DEL provenant de chez Seoul Optodevice Co., LTD (Corée du Sud), de chez Nichia Corporation (Japon), ou encore de chez SunLED Corporation (Etats-Unis).

Selon le procédé de l'invention la culture peut être soumise au rayonnement lumineux pendant un temps suffisant correspondant au moins au temps nécessaire pour que les critères de taux de croissance, de phycocyanine et/ou de caroténoïdes désirés soient remplis. L'homme du métier saura sans expérimentation excessive juger de ce temps nécessaire. Il saura adapter ce temps grâce à ses connaissances du domaine.

Plus particulièrement, les conditions de mixotrophie peuvent être obtenues dans des conditions d'éclairage discontinu et/ou variable au cours du temps.

Par éclairage discontinu, il faut entendre un éclairage ponctué par des périodes d'obscurité. L'éclairage peut être notamment sous forme de flashs. Un flash, au sens de l'invention, est un éclairage lumineux de durée donnée.

Selon l'invention, 3 notions sont à considérer à propos de l'éclairage : la fréquence ou le nombre de flashs par unité de temps, la durée du flash et l'intensité de la lumière émise.

En termes de fréquence selon l'invention, on définit, selon le nombre de flashs par unité de temps utilisé dans le procédé selon l'invention deux types d'éclairage :
- un éclairage à basse fréquence dont le nombre de flashs peut être compris entre environ 2 et 3,6 10⁴ par heure (5,4.10⁻⁴ Hz à 10 Hz), préférentiellement entre 3 et 3,6 10³ par heure (8,3.10⁻⁴ Hz à 1 Hz). On entend bien ici que le nombre de flashs par heure peut prendre toutes les valeurs comprises entre 2 et 36000 sans qu'il soit nécessaire de toutes les citer (2, 3, 4,..., 35598, 35599, 36000) ;
- un éclairage à haute fréquence dont le nombre de flashs peut être compris entre environ 3,6 ×10⁴ et 5,4 × 10⁹ (10 Hz à 1,5.10⁶ Hz) par heure, préférentiellement entre 3,6×10⁵ et 5,4×10⁹ (100 Hz à 1,5.10⁶ Hz). On entend bien ici que le nombre de flashs par heure peut prendre toutes les valeurs comprises entre 3,6 ×10⁵ et 5,4 × 10⁹ sans qu'il soit nécessaire de toutes les citer (36000, 36001, 36002,..., 5399999998, 5399999999, 5400000000).

En termes de durée selon l'invention, quelle que soit la fréquence d'éclairage choisie, la durée du flash peut être comprise entre 1/150000 de seconde et 1799 secondes (29 minutes et 59 secondes).

Bien évidemment lorsque l'on utilise un éclairage à haute fréquence la durée du flash pourra être préférentiellement comprise entre 1/150000 de seconde et 1/10 de seconde.

Et lorsque l'on utilise un éclairage à basse fréquence la durée du flash pourra être préférentiellement comprise entre 1/10 de seconde et 1799 secondes (29 minutes et 59 sec).

En termes d'intensité lumineuse selon l'invention, l'intensité de la lumière apportée sous forme de flashs peut être comprise entre 5 et 5000 µmol. m⁻². s⁻¹, de préférence entre 5 et 500 µmol. m⁻². s⁻¹, ou 50 et 400 µmol. m⁻². s⁻¹, et plus préférentiellement entre 150 et 300 µmol. m⁻². s⁻¹ (1 µmol. m⁻². s⁻¹ correspond à 1 µE m⁻². s⁻¹ (Einstein), unité souvent utilisée dans la littérature).

Selon l'invention le nombre de flashs par heure peut être choisi en fonction de l'intensité et de la durée des flashs (voir ci-dessus).

Selon l'invention les notions de fréquence, de durée et d'intensité lumineuse s'appliquent à l'éclairage tel que le prévoit l'invention, c'est-à-dire à l'éclairage produit par la source lumineuse choisie, avantageusement par une DEL, émettant un rayonnement lumineux de spectre étroit compris entre 400 et 550 nm de préférence 420 et 500 nm, encore plus préférentiellement comprise entre 430 et 480 nm, très préférentiellement centré à 455 nm et pendant les temps considérés selon l'invention.

Une forme préférée de l'invention pourra être un procédé selon l'invention dans lequel l'apport d'éclairage peut être réalisé sous la forme d'une lumière discontinue sous forme de flashs, obtenue avec des DELs émettant un rayonnement présentant un spectre étroit de longueur d'onde comprise entre 400 nm et 550 nm, préférentiellement entre 420 nm et 500 nm, encore plus préférentiellement comprise entre 430 et 480 nm, très préférentiellement de longueur d'onde de 455nm.

Selon un autre mode de réalisation de l'invention, l'éclairage peut être variable, ce qui signifie que l'éclairage n'est pas interrompu par des phases d'obscurité, mais que l'intensité lumineuse varie au cours du temps. Cette variation de l'intensité de lumière peut être régulière ou non, et peut être périodique ou cyclique. Selon l'invention, on peut aussi procéder à un apport lumineux alliant des phases d'éclairage continues et des phases d'éclairage discontinues.

Par éclairage variable, on entend que l'intensité de la lumière varie de manière régulière au moins deux fois par heure.

De tels moyens et méthodes de culture avec éclairage variable ou sous forme de flashes sont décrits dans la demande (WO 2012/035262).

L'éclairage peut présenter, de préférence, des variations d'intensité dont l'amplitude peut être généralement comprise entre 5 µmol. m⁻². s⁻¹ et 5000 µmol. m⁻². s⁻¹, de préférence entre 50 et 1500 µmol. m⁻². s⁻¹, plus préférentiellement entre 50 et 200 µmol. m⁻². s⁻¹.

Selon un mode de réalisation préféré, l'éclairage peut présenter des variations d'intensité dont l'amplitude peut être comprise entre 5 et 1000 µmol. m⁻². s⁻¹, de préférence entre 5 et 400 µmol. m⁻². s⁻¹, ces variations pouvant avoir lieu entre 2 et 3600 fois par heure, de préférence entre 2 et 200 fois par heure.

Ces conditions de culture permettent d'apporter une quantité définie de lumière. Cet apport lumineux peut comporter des phases d'éclairage discontinu et/ou variable, avec des variations d'intensité pouvant avoir des amplitudes identiques ou différentes.

Selon l'invention, les conditions de culture des souches d'ARUs du genre Galdieria pourront être les conditions connues et utilisées dans l'art antérieur pour cultiver les souches retenues, conditions auxquelles une étape d'éclairage aura été ajoutée. Cette étape d'éclairage pourra être ajoutée en début, au milieu ou en fin de culture, le procédé étant alors une combinaison de culture en hétérotrophie et de culture en mixotrophie.

Selon un mode particulier de réalisation de l'invention, l'étape de culture a1) comprend une première étape de culture en hétérotrophie, suivie d'une étape a2) de culture en mixotrophie jusqu'au terme de la culture avant de procéder à l'étape b) de récupération de la biomasse.

Selon un autre mode de réalisation de l'invention, l'ensemble de l'étape a) de culture est réalisée en mode mixotrophe, les conditions d'éclairage pouvant être constantes ou variables dans le temps.

Avantageusement, on utilisera les conditions qui permettront d'obtenir la meilleure productivité en biomasse /et/ou la meilleure concentration en biomasse.

L'homme du métier saura intégrer l'étape d'éclairage selon l'invention dans un procédé connu, afin d'obtenir une biomasse qui réponde aux critères selon l'invention en taux de croissance et en taux de métabolites d'intérêt, particulièrement en taux de caroténoïdes et/ou de phycobiliprotéines, recherchés. A cet égard, on pourra citer les procédés décrits par Wolfgang Gross and Claus Schnarrenberger (*opus cit*.).

Les procédés permettant d'obtenir une productivité en biomasse ou une concentration en biomasse important pourront être privilégiés. Comme exemple de procédé on pourra citer celui décrit par exemple par Graverholt & al. (2007).

Plus particulièrement cette étape pourra être intégrée dans les procédés décrits dans la demande WO 2012/035262.

D'une manière générale selon l'invention, la culture selon le procédé pourra s'effectuer à une température comprise entre 15°C et 47°C, avantageusement entre 22°C et 42°C.

Selon l'invention le procédé de culture peut être utilisé pour cultiver une seule souche d'ARUs du genre Galdieria, ou plusieurs souches du genre Galdieria (au moins 2 espèces).

Le substrat carboné organique, autre que le lactose, contenu le cas échéant dans le milieu de culture peut consister en des molécules complexes ou en un mélange de substrats. Les produits issus de la biotransformation de l'amidon, par exemple à partir de maïs, de blé ou de pomme de terre, notamment les hydrolysats de l'amidon, qui sont constitués de molécules de petite taille, constituent, par exemple, des substrats carbonés organiques adaptés à la culture en mixotrophie des cellules selon l'invention.

Les quantités de sources carbonées utilisées selon le procédé dépendront bien évidement de la souche choisie. L'homme du métier saura là encore sans difficulté adapter les quantités de source carbonée à la souche à cultiver sous forme pure ou en mélange.

Selon un mode de réalisation de l'invention, la source carbonée totale, que ce soit du lactose seul ou du lactose et au moins une autre source de carbone, pourra avoir une concentration comprise entre 0,05 g/L et 200 g/L, de préférence 0,5 g/L et 100 g/L.

Le procédé selon l'invention peut en outre comprendre une étape de récupération des ARUs du genre Galdieria. Ladite récupération des ARUs du genre Galdieria peut être réalisée par toute technique permettant la récupération de la biomasse, notamment les méthodes de filtration, gravimétrique ou sous pression réduite, de décantation, ou bien encore des méthodes de précipitation suivie d'une filtration gravimétrique.

L'invention concerne également la biomasse susceptible d'être obtenue par l'une quelconque des variantes du procédé selon l'invention.

On entend avantageusement par "biomasse" selon l'invention un ensemble de cellules de microorganismes produits par leur culture, cellules qui peuvent avoir conservé ou non leur intégrité physique. On comprend donc que ladite biomasse peut comprendre une quantité de cellules de microorganismes dégradées allant de 0% à 100%. Par "dégradée" on entend que l'intégrité physique desdites cellules de microorganismes a pu être altérée comme par exemple des microorganismes lysés, résultant par exemple d'un procédé d'homogénéisation ou lyse enzymatique. Une fois produite, cette biomasse pourra être brute, juste séparée de son milieu de culture, séchée ou non, dégradée ou non.

La biomasse, selon qu'elle soit séchée ou non, totalement ou en partie, peut comprendre un taux d'humidité de 1% à 90%.

Selon un premier mode de réalisation, la biomasse a un taux d'humidité de 70% à 90 %, préférentiellement 80% à 85 %. C'est en particulier le cas lorsqu'elle est essentiellement constituée de microorganismes industriels, optimisés et cultivés, après filtration du mout de fermentation pour séparer les microorganismes cultivés du milieu de culture, avant séchage.

Selon un autre mode de réalisation de l'invention, la biomasse est séchée, totalement ou en partie et présente un taux d'humidité de 1% à 10%, préférentiellement de 2% à 7%. Selon l'invention ladite biomasse peut présenter une densité en ARUs du genre Galdieria comprise entre 20 et 200 g/l de matière sèche, préférentiellement entre 90 et 150 g/l de matière sèche.

Selon l'invention ladite biomasse peut présenter une quantité de protéines comprise entre 25% et 60%, voire jusqu'à 70 %, de préférence entre 30% et 55%, plus préférentiellement entre 40% et 50% du poids de matière sèche. Le dosage de l'azote et le calcul de la teneur en protéines brutes sont réalisés selon la méthode de digestion en bloc et distillation à la vapeur (NF EN ISO 5983-2).

Selon l'invention ladite biomasse peut présenter une teneur intracellulaire en phycobiliprotéines (phycocyanine et allophycocyanine) comprises entre 1 et 250 mg/g de matière sèche, préférentiellement entre 20 et 150 mg/g de matière sèche.

Selon l'invention encore ladite biomasse peut présenter une teneur intracellulaire en phycocyanine comprise entre 0,5 et 100 mg/g de matière sèche, préférentiellement entre 10 et 40 mg/g de matière sèche.

La biomasse pourra être conditionnée pour son stockage ou pour son utilisation en tant que telle, par exemple comme complément alimentaire ou aliment pour l'alimentation humaine ou animale.

Le tourteau susceptible d'être obtenu après extraction de la phycocyanine à partir de la biomasse d'ARUs du genre Galdieria susceptible d'être obtenue par le procédé selon l'invention peut être utilisé comme complément alimentaire riche en protéines et caroténoïdes, dans l'alimentation humaine ou animale.

Selon l'invention ladite biomasse peut présenter une teneur intracellulaire en caroténoïdes comprise entre 0,1 et 10 mg/g de matière sèche, avantageusement entre 0,250 et 1 mg/g de matière sèche.

Les ARUs présentent un potentiel d'utilisation important dans beaucoup de domaines dont on citera par exemple, l'alimentation humaine ou animale, la cosmétique, la médecine.

Selon l'invention, ladite biomasse d'ARUs du genre Galdieria susceptible d'être obtenue selon l'invention peut être utilisée après récolte soit directement, éventuellement séchée, soit après transformation. En particulier ladite biomasse peut être utilisée sous la forme de farines entrant dans des compositions alimentaires ou sous forme de compléments alimentaires.

La biomasse d'ARUs du genre Galdieria susceptible d'être obtenue selon l'invention peut être transformée en farine selon tout procédé connu de l'homme du métier. On peut ainsi envisager par exemple que les ARUs du genre Galdieria puissent être séparées du milieu de culture, lysées et réduites en particules fines (diamètre moyen de 10 microns), puis séchées.

L'invention concerne également toute utilisation de la biomasse d'ARUs du genre Galdieria susceptible d'être obtenue selon l'invention dans tout domaine connu d'utilisation des ARUs, particulièrement, l'alimentation humaine ou animale, la cosmétique, la médecine. Dans les domaines de l'alimentation humaine ou animale et de la cosmétique il s'agit bien évidement d'utilisations non thérapeutiques qui s'adresse à des animaux ou des êtres humains sains.

La biomasse obtenue après culture de ARUs du genre Galdieria selon le procédé de l'invention peut permettre d'obtenir en particulier une farine riche en agents antioxydants, en particulier en caroténoïdes (particulièrement zéaxanthine et ß-carotènes) en teneurs comprises entre 0,1 et 10 mg/g de matière sèche, avantageusement entre 0,25 et 1 mg/g de matière sèche dont en particulier de la zéaxanthine en une teneur comprise entre 0,05 et 5 mg/g de matière sèche, avantageusement entre 0,1 et 1 mg/g de matière sèche, et/ou du ß-carotène en une teneur 0,05 et 5 mg/g de matière sèche, avantageusement entre 0,1 et 1 mg/g de matière sèche, répondant à un besoin en particulièrement dans l'industrie alimentaire, parce que plus appétentes, ayant meilleur goût, apportant des antioxydants en quantité importante et pouvant être utilisable dans l'alimentation animale ou humaine.

L'invention concerne donc une farine susceptible d'être obtenue après transformation de la biomasse en ARUs du genre Galdieria susceptible d'être obtenue par le procédé selon l'invention.

Quelle que soit la forme d'utilisation du produit susceptible d'être obtenu par le procédé selon l'invention (biomasse native ou transformée), ledit produit peut être utilisé pur ou mélangé à d'autres ingrédients classiquement utilisés, particulièrement dans des utilisations non thérapeutiques en alimentation ou en cosmétique.

L'invention concerne également tout produit pouvant comprendre au moins de la biomasse d'algues susceptible d'être obtenue selon l'invention. L'invention concerne aussi tout produit pouvant comprendre au moins de la farine issue de la transformation de la biomasse d'algues susceptible d'être obtenue selon l'invention.

En mixotrophie, la culture de *Galdieria* sur perméat de lait comme source de lactose prend une couleur blanche jaune et perd sa pigmentation verte bleue. Plusieurs études ont démontré que plusieurs étapes clés de la voie de synthèse de phycocyanine et de la chlorophylle sont induites par la lumière et d'autres réprimées en présence de substrats organique dans le milieu comme le glucose (Stadnichuck & al., 1998). Sur perméat de lait l'effet inhibiteur du lactose est encore plus prononcé et aboutit à une perte totale de chlorophylle et de phycocyanine. Toutefois, il existe des mutants naturels (Gross *et al.,* 1995; Sloth & al. 2006) capable de produire de la phycocyanine même en condition hétérotrophe sur glucose. Il est donc envisageable d'utiliser une telle souche pour produire de la phycocyanine en hétérotrophie ou en mixotrophie, et du lactose comme source principale de carbone.

Selon l'invention les phycobiliprotéines, et particulièrement la phycocyanine, produites par ladite biomasse peuvent être extraites pour être utilisées par exemple dans l'alimentation ou encore comme colorant. L'extraction des phycobiliprotéines, et particulièrement de la phycocyanine, à partir de ladite biomasse peut se faire selon toute technique d'extraction connue de l'homme du métier comme par exemple celle décrite par Moon & al. (2014) ou par Jaouen & al. (1999) ou dans la demande FR 2 789 399.

L'invention concerne également l'utilisation de la phycocyanine susceptible d'être obtenue selon le procédé de l'invention, dans l'alimentation, animale ou humaine, comme complément alimentaire, ou encore comme colorant, particulièrement comme colorant alimentaire.

On notera que dans le présent texte, telles qu'elles peuvent être utilisées dans la description et les revendications, les formes singulières "un", "une", "le" et "la" englobent les références à leurs pluriels, sauf si le contexte en convient clairement autrement.

### DESCRIPTION DES FIGURES

La Figure 1 représente la croissance de la souche de *Galdieria sulphuraria UTEX 2919* sur perméat de lait en Erlen.
La Figure 2 représente la croissance de la souche de *Galdieria sulphuraria UTEX 2919* sur perméat de lait en poudre réhydraté en fermenteur.
La Figure 3 représente la croissance de la souche de *Galdieria sulphuraria UTEX 2919* sur perméat de lait liquide en fermenteur.

D'autres caractéristiques et avantages de l'invention pourront apparaitre à la lecture des exemples qui suivent et qui illustrent la présente demande sans toutefois la limiter ainsi que des figures annexées dans lesquelles

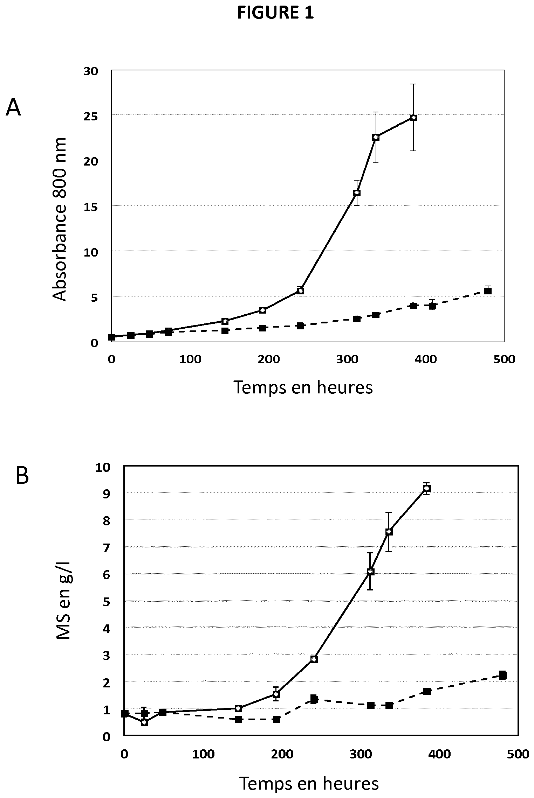

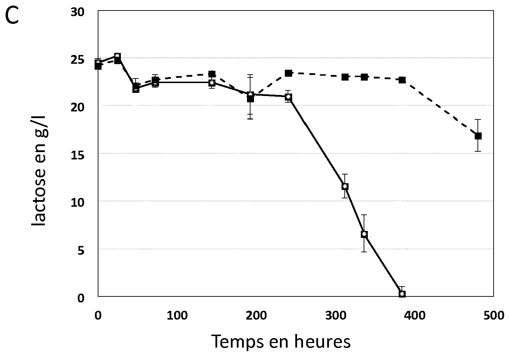

La Figure 1 illustre la croissance de la souche de *Galdieria sulphuraria UTEX 2919* sur perméat de lait en Erlen, avec en (A) le suivi de croissance par absorbance à 800 nm au cours du temps ; en (B) le suivi de croissance par masse sèche au cours du temps ; avec en (C) le suivi de consommation du lactose dans le milieu au cours du temps. La concentration en lactose dans le milieu est mesurée par HPLC. Chaque courbe (-O-) croissance de la souche en condition mixotrophe ; (--■--) croissance de la souche en condition hétérotrophe est une moyenne de trois essais.

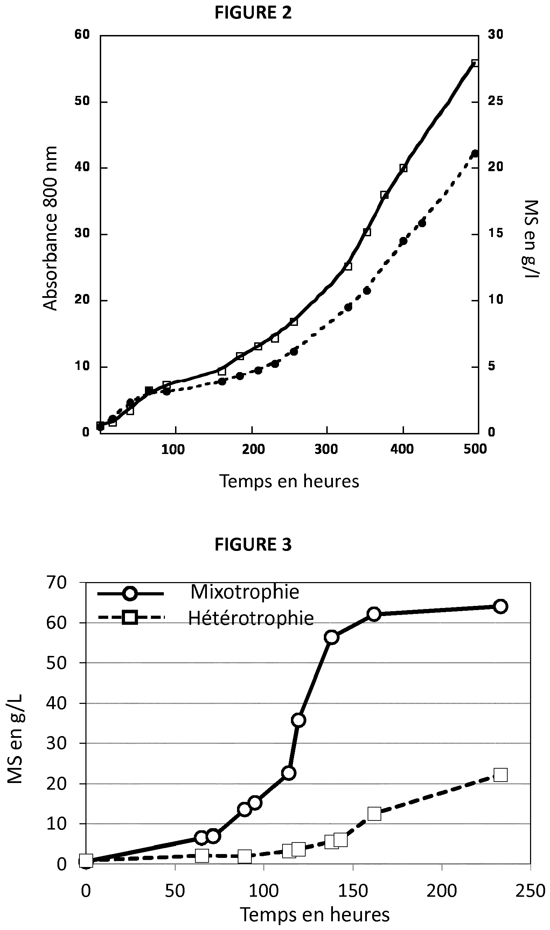

La Figure 2 illustre la croissance de la souche de *Galdieria sulphuraria UTEX* 2919 sur perméat de lait en poudre réhydraté, en Fermenteur ; (-□-) suivi de croissance par absorbance à 800 nm au cours du temps ; (--●--) suivi de croissance par masse sèche au cours du temps.

La Figure 3 illustre la croissance de la souche de *Galdieria sulphuraria UTEX* 2919 sur perméat de lait liquide en Fermenteur ; (--□--) suivi de croissance en hétérotrophie par masse sèche au cours du temps ; (-○-) suivi de croissance en mixotrophie par masse sèche au cours du temps.

### EXEMPLES

### Exemple 1 : Suivi de croissance de la souche Galdieria sulphuraria sur perméat de lait en Erlenmeyer.

La croissance de la souche est réalisée par mesure de d'absorbance à 800 nm au court du temps. Le milieu de culture est un milieu classique pour cette souche, si ce n'est que le milieu comprend du perméat de lait à 30 g/l ce qui correspond à peu près à 25 g/L de lactose.

### Matériel et méthodes

**Souche :** *Galdieria sulphuraria (aussi appelée Cyanidium caldarium) UTEX#2919*

### Milieu de culture :

30 g/L de perméat de lait (environ 25 g/L de lactose), 8 g/L (NH₄)₂SO₄, 1g/L KH2PO4, 716mg/L MgSO₄, 44mg/L CaCl₂, 3 mL/L de solution stock Fe-EDTA (FeSO₄ à 6,9g/L et d'EDTA-Na₂ à 9,3g/L ) et 4 ml/L de solution de trace métal (3,09g/L EDTA-Na₂ ; 0,080g/L CuSO₄,5H₂O ; 2,860g/L H₃BO₃; 0,040g/L NaVO₃, 4H₂O ; 1,820g/L MnCl₂ ; 0,040g/L CoCl₂,6H₂O ; 0,220g/L ZnSO₄,7H₂O ; 0,017g/L Na₂SeO₃ ; 0,030g/L (NH₄)₆Mo₇O₂₄, 4H₂O).

### Conditions de culture :

La culture est réalisée sur table d'agitation (140 t/min) en enceinte thermostatée (37 °C), en milieu comme décrit ci-dessus en présence d'une source de lumière Le temps de culture a été compris entre 200 et 500 heures.

### Suivi des cultures :

Le suivi de croissance se fait par absorbance au spectrophotomètre à 800 nm. La concentration en biomasse totale est suivie par mesure de la masse sèche (filtration sur filtre GF/F, Whatman, puis séchage en étuve, à 105°C, pendant 24 h minimum avant pesée).

Les résultats sont présentés à la figure 1.

### Résultats

Le suivi de croissance montre que l'on atteint des DO importantes et que la totalité du lactose a été consommée. Le dosage du lactose a été réalisé par HPLC (Shimatsu) en mode isocractique H₂SO₄ 5 mM et détection RI (Refractive Index). Lors de ces analyses par HPLC aucune trace de glucose ou de galactose, synonyme d'hydrolyse du lactose, n'a pu être détectée dans le milieu.

Les résultats des analyses du contenu en acide aminé de la souche sont décrits dans les tableaux 1A, 1B et 1C suivants :

**Tableau 1 (A) : Contenu en acide aminé de la souche ayant poussée sur lactose en mixotrophie. Quantité en acides aminés exprimée pour 100 g de matière sèche.**

| *1A* | | | | | | | | |
|---|---|---|---|---|---|---|---|---|
| ASP | GLU | ALA | ARG | CYS* | GLY | HIS | ILE | LEU |
| 3,73 | 7,16 | 2,37 | 2,5 | 1,04 | 2,00 | 0,958 | 2,21 | 3,5 |
| LYS | MET | PHE | PRO | SER | THR | TYR | VAL | TRP |
| 3,25 | 1,04 | 2,06 | 2,31 | 3,06 | 2,74 | 2,85 | 2,97 | 0,72 |
| TOTAL : 46,47 | | * : Cystéine + Cystine ; Valeurs données en g/100g de Matière Sèche | | | | | | |

**Tableau 1(B) : Contenu en acide aminé de la souche ayant poussée sur lactose en mixotrophie : Aminoscores comparant l'apport journalier recommandé par la FAO et les quantités apportés par les souches de cette étude (mg/g de protéines).**

| *1B* | mg/g N*6,25 (FAO) | mg/g N*6,25 UTEX 2919 |
|---|---|---|
| HIS | 16 | 18,66 |
| ILE | 30 | 43,04 |
| LEU | 61 | 68,16 |
| LYS | 48 | 63,29 |
| MET + CYS | 23 | 40,51 |
| PHE + TYR | 41 | 95,62 |
| THR | 25 | 53,36 |
| TRP | 6,6 | 14,02 |
| VAL | 40 | 57,84 |

**Tableau 1(C) : Contenu en acide aminé de la souche ayant poussée sur lactose en mixotrophie : Estimation de la quantité de protéines (N*6,25) par mesure d'azote total.**

| *1C* | N total | N*6,25 |
|---|---|---|
| Kjeldahl | 8,217 | 51,35 |

Le contenu protéique, estimé par la méthode de Kjeldahl, a été estimé à 51,35 % en utilisant le facteur N*6,25.

L'apport journalier en acides aminés essentiels apporté par la consommation de 100 g de protéines de *Galdieria sulphuraria,* produite dans les conditions décrites auparavant, est supérieur aux recommandations faites par la FAO

### Exemple 2 : Suivi de croissance de la souche Galdieria sulphuraria sur perméat de lait en poudre réhydraté en Fermenteur.

La croissance de la souche est réalisée par mesure de d'absorbance à 800 nm au court du temps. Le milieu de culture est un milieu classique pour cette souche, si ce n'est que le milieu comprend du perméat de lait à 30 g/l ce qui correspond à peu près à 25 g/L de lactose, 3,12 g/L de citrate, et 0,76g/L de lactate..

### Matériel et méthodes

**Souche :** *Galdieria sulphuraria (aussi appelée Cyanidium caldarium) UTEX#2919*

### Milieu de culture :

30 g/L de perméat de lait (environ 25 g/L de lactose, 3,12 g/L de citrate, et 0,76g/L de lactate), 8 g/L (NH₄)₂SO₄, 1g/L KH2PO4, 716mg/L MgSO₄, 44mg/L CaCl₂, 3 mL/L de solution stock Fe-EDTA (FeSO₄ à 6,9g/L et d'EDTA-Na₂ à 9,3g/L ) et 4 ml/L de solution de trace métal (3,09g/L EDTA-Na₂ ; 0,080g/L CuSO₄,5H₂O ; 2,860g/L H₃BO₃ ; 0,040g/L NaVO₃, 4H₂O ; 1,820g/L MnCl₂ ; 0,040g/L CoCl₂,6H₂O ; 0,220g/L ZnSO₄,7H₂O ; 0,017g/L Na₂SeO₃ ; 0,030g/L (NH₄)₆Mo₇O₂₄, 4H₂O).

### Conditions de culture :

Les cultures sont réalisées dans des réacteurs de 1 à 2 L de volume utile avec automates dédiés et supervision par station informatique. Le pH de la culture est régulé via l'ajout de base (solution d'ammoniaque 14% (w NH3/w) et/ou d'acide (solution d'acide sulfurique 4N). La température de culture est fixée à 42 °C. L'agitation est réalisée grâce à 3 mobiles d'agitation : 1 turbine Rushton à 6 pâles droites positionnée à l'extrémité inférieure de l'arbre d'agitation au-dessus du "sparger" et 2 hélices tripâles HTPG2 placés sur l'arbre d'agitation. La pression en oxygène dissous dans la phase liquide est régulée dans le milieu tout au long de la culture, par la vitesse de rotation de l'arbre d'agitation (250-1800 t/min), le débit de ventilation par l'air et/ou d'oxygène. Les paramètres de régulation, intégrés dans l'automate de supervision, permettent de maintenir une pression partielle en oxygène dissous dans la phase liquide comprise entre 5 et 30 % de la valeur de saturation par l'air dans des conditions identiques de température, de pression et de composition du milieu. Le temps de culture a été compris entre 200 et 500 heures. Des ajouts de solution nutritive contenant du perméat de lait à 100 g/l sont réalisés au cours du temps pour maintenir une concentration en lactose dans le fermenteur comprise entre 10 et 20 g/l.

Le suivi de croissance se fait par absorbance au spectrophotomètre à 800 nm. La concentration en biomasse totale est suivie par mesure de la masse sèche (filtration sur filtre GF/F, Whatman, puis séchage en étuve, à 105°C, pendant 24 h minimum avant pesée).

Les résultats de cet essai sont illustrés par la Figure 2.

### Résultats

Le suivi de croissance montre que l'on atteint des DO importantes et une masse sèche de plus de 20 g par litre de culture, avec du lactose comme seule source de carbone.

### Exemple 3 : Suivi de croissance de la souche Galdieria sulphuraria sur perméat de lait liquide en Fermenteur.

La croissance de la souche est réalisée par mesure de d'absorbance à 800 nm au court du temps. Le milieu de culture est un milieu classique pour cette souche, si ce n'est que le milieu comprend du perméat de lait liquide contenant à peu près à 81 g/L de lactose, 11,9 g/L de citrate , 2,8 g/L de lactate.

### Matériel et méthodes

**Souche :** *Galdieria sulphuraria (aussi appelée Cyanidium caldarium) UTEX#2919*

### Milieu de culture :

Perméat de lait (environ 25 g/L de lactose, 3,65 g/L de citrate, et 0,86 g/L de lactate), 8 g/L (NH₄)₂SO₄, 1g/L KH2PO4, 716mg/L MgSO₄, 44mg/L CaCl₂, 3 mL/L de solution stock Fe-EDTA (FeSO₄ à 6,9g/L et d'EDTA-Na₂ à 9,3g/L ) et 4 ml/L de solution de trace métal (3,09g/L EDTA-Na₂ ; 0,080g/L CuSO₄,5H₂O ; 2,860g/L H₃BO₃; 0,040g/L NaVO₃, 4H₂O ; 1,820g/L MnCl₂ ; 0,040g/L CoCl₂,6H₂O ; 0,220g/L ZnSO₄,7H₂O ; 0,017g/L Na₂SeO₃ ; 0,030g/L (NH₄)₆MO₇O₂₄, 4H₂O).

### Conditions de culture :

Les cultures sont réalisées dans des réacteurs de 1 à 2 L de volume utile avec automates dédiés et supervision par station informatique. Le pH de la culture est régulé via l'ajout de base (solution d'ammoniaque 14% (w NH3/w) et/ou d'acide (solution d'acide sulfurique 4N). La température de culture est fixée à 42 °C. L'agitation est réalisée grâce à 3 mobiles d'agitation : 1 turbine Rushton à 6 pâles droites positionnée à l'extrémité inférieure de l'arbre d'agitation au-dessus du "sparger" et 2 hélices tripâles HTPG2 placés sur l'arbre d'agitation. La pression en oxygène dissous dans la phase liquide est régulée dans le milieu tout au long de la culture, par la vitesse de rotation de l'arbre d'agitation (250-1800 t/min), le débit de ventilation par l'air et/ou d'oxygène. Les paramètres de régulation, intégrés dans l'automate de supervision, permettent de maintenir une pression partielle en oxygène dissous dans la phase liquide comprise entre 5 et 30 % de la valeur de saturation par l'air dans des conditions identiques de température, de pression et de composition du milieu. Le temps de culture a été compris entre 200 et 250 heures. Des ajouts de solution nutritive contenant du perméat de lait à 81 g/l sont réalisés au cours du temps pour maintenir une concentration en lactose dans le fermenteur comprise entre 10 et 20 g/l.

En Mixotrophie, les cellules sont éclairées par un système de contre-pales lumineuses équipées d'un système DEL, délivrant une lumière blanche en continue d'une puissance de 3 Watts.

Le suivi de croissance se fait par absorbance au spectrophotomètre à 800 nm. La concentration en biomasse totale est suivie par mesure de la masse sèche (filtration sur filtre GF/F, Whatman, puis séchage en étuve, à 105°C, pendant 24 h minimum avant pesée).

### Résultats

Les résultats de cet essai sont illustrés par la Figure 3.

Le suivi de croissance montre clairement que la croissance sur perméat en mixotrophie est plus importante qu'en hétérotrophie. Cela ce traduit également par des vitesses moyennes de consommation en substrats organiques contenus dans le milieu beaucoup plus importante (Tableau 2A et 2B). Les résultats contenus dans ce tableau montre également que la souche UTEX 2919 à la capacité de consommer les acides organiques, comme l'acide citrique et l'acide lactique, contenu dans le perméat de lait dans des proportions non négligeable.

**Tableau 2 (A) : consommation de substrats organiques contenu dans le perméat de lait, en mixotrophie.**

| **UTEX 2919 Mixotrophie sur Perméat de lait** | | | |
|---|---|---|---|
| | **Quantité totale ajoutée (g)** | **Consommation moyenne en g/L/h** | **Pourcentage consommé (%)** |
| Lactose | 94 | 0,27 | 100 |
| Lactate | 3,4 | 0,009 | 98 |
| Citrate | 14,4 | 0,032 | 79 |

**Tableau 2B : Consommation de substrats organiques contenu dans le perméat de lait, en hétérotrophie.**

| **UTEX 2919 Hétérotrophie sur Perméat de lait** | | | |
|---|---|---|---|
| | **Quantité totale ajoutée (g)** | **Consommation moyenne en g/L/h** | **Pourcentage consommé (%)** |
| Lactose | 34 | 0,17 | 100 |
| Lactate | 1,6 | 0,004 | 58 |
| Citrate | 4,5 | 0,009 | 42 |

En mixotrophie, la totalité totalité du lactose est consommée en fin de croissance, ainsi que 98 % et 80 % du citrate, les vitesses de consommation de substrats sont entre 1,5 et 3,5 fois plus rapides.

### RÉFÉRENCES

- Graverholt & al. (2007), Appl. Microbiol. Biotechnol. (2007) 77:69-75
- Gross et al., (1995), Plant and Cell Physiology 36, no. 4 (June 1, 1995): 633-38
- Jaouen & al., (1999), Biotechnology Techniques 13, no. 12 (December 1999): 877-81
- Moon & al., (2014), "Isolation and Characterization of Thermostable Phycocyanin from Galdieria Sulphuraria" 31 (2014): 1-6
- Sloth et al., (2006), Enzyme and Microbial Technology 38, no. 1-2 (January 3, 2006): 168-75
- Stadnichuck et al., (1998) Plant Science 136, no. 1 (August 7, 1998): 11-23
- Tischendorf & al. (2007), European Journal of Phycology, vol. 42, no. 3, 1 amoût 2007, 243-251
- EP 2 730 647
- FR 2 789 399
- WO 2012/035262
- WO 2014/074769
- WO 2012/175866

## Revendications

1. Procédé de production d'une biomasse d'algues rouges unicellulaires (ARUs) du genre Galdieria, comprenant les étapes suivantes :
a) de culture desdites algues rouges unicellulaires (ARUs)du genre Galdieria dans un milieu comprenant une source de carbone, et
b) de récupération de la biomasse produite comprenant lesdites algues rouges unicellulaires (ARUs) du genre Galdieria à partir du milieu de culture,
**caractérisé en ce que** le milieu comprend au moins un perméat lacté comprenant du lactose comme source de carbone.

2. Procédé selon la revendication 1, **caractérisé en ce que** le lactose est la seule source de carbone.

3. Procédé selon l'une des revendications 1 ou 2, **caractérisé en ce que** le perméat lacté est choisi parmi le perméat de lait, le perméat de sérum, le babeurre et leurs mélanges.

4. Procédé selon l'une quelconque des revendications 1 à 3, **caractérisé en ce que** le lactose est à une concentration initiale dans le milieu de culture comprises entre 0,1 g/L et 150 g/L, préférentiellement comprise entre 10 g/L et 80 g/L.

5. Procédé selon l'une quelconque des revendications 1 à 4, **caractérisé en ce qu'**il comprend au moins une étape d'éclairage.

6. Procédé selon l'une quelconque des revendications 1 à 5, **caractérisé en ce que** les ARUs du genre *Galdieria* sont choisies parmi les espèces *Galdieria daedala, Galdieria maxima, Galdieria partita* et *Galdieria sulphuraria.*

7. Procédé selon l'une des revendications 1 à 6, **caractérisé en ce que** les ARU du genre Galdierie asont choisis parmi l'espèce *Galdieria sulphuraria.*

8. Procédé selon l'une quelconque des revendications 1 à 7, **caractérisé en ce que** le milieu de culture comprend le lactose du perméat lacté et une autre source de carbone.

9. Procédé selon l'une quelconque des revendications 1 à 8, **caractérisé en ce que** la source carbonée autre que le lactose est choisie parmi le glucose, le saccharose, l'acétate.

10. Procédé selon l'une quelconque des revendications 1 à 9, **caractérisé en ce que** la source carbonée totale est à une concentration dans le milieu initial comprise entre 0,05 g/L et 200 g/L, de préférence 0,5 g/L et 100 g/L.

11. Procédé selon l'une des revendications 1 à 10, **caractérisé en ce que** la biomasse récupérée est transformée en farine.

12. Procédé selon l'une des revendications 1 à 10, **caractérisé en ce que** la biomasse récupérée est conditionnée pour son utilisation comme complément alimentaire ou aliment pour l'alimentation humaine ou animale.

13. Procédé selon l'une quelconque des revendications 1 à 10, **caractérisé en ce qu'**il comprend une étape c) d'extraction des phycobiliprotéines, en particulier des phycocyanines, de la biomasse récupérée.

14. Procédé selon la revendication 12, **caractérisé en ce que** l'on récupère le tourteau de biomasse après extraction des phycobilliprotéines.

## Patentansprüche

1. Verfahren zur Herstellung einer Biomasse aus einzelligen Rotalgen (URAs) der Gattung Galdieria, umfassend die folgenden Schritte:
a) Kultivieren der einzelligen Rotalgen (URAs) der Gattung Galdieria in einem Medium, das eine Kohlenstoffquelle umfasst, und
b) Rückgewinnen der erzeugten Biomasse, welche die einzelligen Rotalgen (URAs) der Gattung Galdieria umfasst, ausgehend von dem Kulturmedium,
**dadurch gekennzeichnet, dass** das Medium mindestens ein Molkereipermeat umfasst, das Laktose als Kohlenstoffquelle umfasst.

2. Verfahren nach Anspruch 1, **dadurch gekennzeichnet, dass** die Laktose die einzige Kohlenstoffquelle ist.

3. Verfahren nach einem der Ansprüche 1 oder 2, **dadurch gekennzeichnet, dass** das Molkereipermeat aus Permeat von Milch, Permeat von Serum, Buttermilch und Mischungen daraus ausgewählt wird.

4. Verfahren nach einem der Ansprüche 1 bis 3, **dadurch gekennzeichnet, dass** die Laktose in dem Kulturmedium in einer Ausgangskonzentration von zwischen 0,1 g/l und 150 g/l, vorzugsweise zwischen 10 g/l und 80 g/l, vorliegt.

5. Verfahren nach einem der Ansprüche 1 bis 4, **dadurch gekennzeichnet, dass** es mindestens einen Beleuchtungsschritt umfasst.

6. Verfahren nach einem der Ansprüche 1 bis 5, **dadurch gekennzeichnet, dass** die URAs der Gattung *Galdieria* aus den Arten *Galdieria daedala, Galdieria maxima, Galdieria partita* und *Galdieria sulphuraria* ausgewählt werden.

7. Verfahren nach einem der Ansprüche 1 bis 6, **dadurch gekennzeichnet, dass** die URAs der Gattung Galdieria aus der Art *Galdieria sulphuraria* ausgewählt werden.

8. Verfahren nach einem der Ansprüche 1 bis 7, **dadurch gekennzeichnet, dass** das Kulturmedium Laktose des Molkereipermeats und eine weitere Kohlenstoffquelle umfasst.

9. Verfahren nach einem der Ansprüche 1 bis 8, **dadurch gekennzeichnet, dass** die weitere kohlenstoffhaltige Quelle neben Laktose aus Glucose, Saccharose und Acetat ausgewählt wird.

10. Verfahren nach einem der Ansprüche 1 bis 9, **dadurch gekennzeichnet, dass** die gesamte kohlenstoffhaltige Quelle in dem Ausgangsmedium in einer Konzentration von zwischen 0,05 g/l und 200 g/l, vorzugsweise 0,5 g/l und 100 g/l, vorhanden ist.

11. Verfahren nach einem der Ansprüche 1 bis 10, **dadurch gekennzeichnet, dass** die rückgewonnene Biomasse zu Mehl verarbeitet wird.

12. Verfahren nach einem der Ansprüche 1 bis 10, **dadurch gekennzeichnet, dass** die rückgewonnene Biomasse für ihre Verwendung als Nahrungsergänzung oder Lebensmittel zur Ernährung von Menschen oder Tieren aufbereitet wird.

13. Verfahren nach einem der Ansprüche 1 bis 10, **dadurch gekennzeichnet, dass** es einen Schritt c) des Extrahierens der Phycobiliproteine, insbesondere der Phycocyanine, von der wiedergewonnenen Biomasse umfasst.

14. Verfahren nach Anspruch 12, **dadurch gekennzeichnet, dass** der Biomassekuchen nach des Extrahierens der Phycobiliproteine wiedergewonnen wird.

## Claims

1. Method for producing a biomass of unicellular red algae (URAs) of the genus Galdieria, comprising the following steps:
a) culturing said unicellular red algae (URAs) of the genus Galdieria in a medium comprising a carbon source, and
b) recovering the produced biomass comprising said unicellular red algae (URAs) of the genus Galdieria from the culture medium,
**characterised in that** the medium comprises at least one lacteal permeate comprising lactose as carbon source.

2. Method according to claim 1, **characterised in that** lactose is the only carbon source.

3. Method according to one of claims 1 or 2, **characterised in that** the lacteal permeate is chosen among milk permeate, whey permeate, buttermilk and the mixtures thereof.

4. Method according to any one of claims 1 to 3, **characterised in that** lactose is at an initial concentration in the culture medium comprised between 0.1 g/L and 150 g/L, preferably comprised between 10 g/L and 80 g/L.

5. Method according to any one of claims 1 to 4, **characterised in that** it comprises at least one lighting step.

6. Method according to any one of claims 1 to 5, **characterised in that** the URAs of the genus *Galdieria* are chosen from among the species *Galdieria daedala, Galdieria maxima, Galdieria partita* and *Galdieria sulphuraria.*

7. Method according to one of claims 1 to 6, **characterised in that** the URA of the genus Galdieria are chosen from the species *Galdieria sulphuraria.*

8. Method according to any one of claims 1 to 7, **characterised in that** the culture medium comprises lactose from the lacteal permeate and another carbon source.

9. Method according to any one of claims 1 to 8, **characterised in that** the carbon source other than lactose is chosen from glucose, sucrose and acetate.

10. Method according to any one of claims 1 to 9, **characterised in that** the total carbon source is at a concentration in the initial medium comprised between 0.05 g/L and 200 g/L, preferably between 0.5 g/L and 100 g/L.

11. Method according to one of claims 1 to 10, **characterised in that** the recovered biomass is transformed into flour.

12. Method according to one of claims 1 to 10, **characterised in that** the recovered biomass is packaged for use thereof as a food supplement or foodstuff for human or animal food.

13. Method according to any one of claims 1 to 10, **characterised in that** it comprises a step c) of extracting phycobiliproteins, in particular phycocyanins, from the recovered biomass.

14. Method according to claim 12, **characterised in that** the biomass cake is recovered after extraction of the phycobiliproteins.
